# EUROPEAN PATENT APPLICATION

(11) **EP 2 402 013 A1**
(43) Date of publication of application: **04.01.2012**
(21) Application number: 11182971.9
(22) Date of filing: 21.11.2007
(51) Int. Cl.: A61K 31/519, A61K 39/00, A61K 31/505, A61K 45/06, A61P 29/00

(54) **Methods of Treating Chronic Inflammatory Diseases using a GM-CSF Antagonist**

(30) Priority: 21.11.2006 US 860780 P; 21.02.2007 US 902742 P
(62) Divisional of application: 07868831.4
(71) Applicant: Kalobios Pharmaceuticals, Inc., Palo Alto, CA 94304 (US)
(72) Inventor: Bebbington, Christopher R, San Mateo, CA California CA 94402 (US); Yarranton, Geoffrey T, Burlingame, CA California CA 94010 (US)
(74) Representative: Campbell, Patrick John Henry

(57) **Abstract**

The invention is based on the discovery that GM-CSF antagonists can be used for the treatment of chronic inflammatory disease, such as rheumatoid arthritis. Accordingly, the invention provides methods of administering a GM-CSF antagonist, *e.g.*, a GM-CSF antibody, and an anti-folate compounds, *e.g.*, methotrexate, to a patient that has RA and pharmaceutical compositions comprising such antagonists.

## Description

### CROSS-REFERENCE TO RELATED APPLICATIONS

This application claims benefit of U.S. provisional application no. 60/860,780, filed November 21, 2006; and U.S. provisional application no. 60/902,742, filed February 21, 2007, each of which applications is herein incorporated by reference.

### BACKGROUND OF THE INVENTION

Rheumatoid arthritis (RA) is a chronic and typically progressive inflammatory disease that affects up to 1% of the adult population worldwide (Gabriel, Rheum Dis Clin North Am 27:269-81, 2001). Current recommendations for treatment of RA include early treatment with disease modifying anti-rheumatic drugs (DMARDs) after the diagnosis has been established. Non-steroidal anti-inflammatory drugs (NSAIDs), and until recently, COX-2 inhibitors have been widely used while waiting to confirm the diagnosis or later in the course of the disease in conjunction with DMARDs. Methotrexate is the most widely used DMARD, but other agents, including hydroxychloroquine, sulfasalazine, gold, minocycline, and leflunomide, are also prescribed. Corticosteroids may be used in combination with DMARDs, but in general, only low doses are used to minimize adverse events (O'Dell, New Engl. J. Med. 350:2591-2603, 2004).

Several new biological drugs have recently been approved for RA treatment. Etanercept (Enbrel ®), blocks Tumour Necrosis Factor alpha (TNF-α); infliximab and adalimumab (Remicade® and Humira®, respectively) block TNF-α and TNF-β; and Anakinra (Kineret®) is an inhibitor of IL-1. These agents act rapidly and have been shown to be disease modifying (slow joint/bone erosion) (Olsen & Stein, New Engl. J. Med 350:2167-2179, 2004). However, some problems remain with these therapies. Some patients do not achieve an adequate response to the TNF inhibitors. Furthermore, in some patients, the therapeutic benefit of the TNF inhibitor is lost over time. Blocking the TNF pathway has also been associated with reactivation of tuberculosis as well as increased risk of severe infections, demyelination, and lymphoma, although RA patients are at higher risk for lymphoma than the general population. Anakinra has a short half-life and must be given as a daily injection and hence, is used less frequently than the longer acting TNF inhibitors as a first line biological therapy.

Recent data on the use of rituximab (Mabthera®), a monoclonal anti-CD20 antibody, in combination with methotrexate in patients with RA has shown benefit over an extended period of time after two infusions of the antibody (Edwards et al., New Engl. J. Med 350: 2572-2581, 2004).

Methotrexate is used as a DMARD to treat RA and other inflammatory arthritic diseases and autoimmune indications, including psoriasis and systemic lupus erythemaotosus. Methotrexate is particularly effective for treating psoriatic arthritis and juvenile idiopathic arthritis. The drug is also used in chemotherapy of cancer at higher doses than are recommended for the treatment of inflammatory arthritis. When used in chemotherapy, methotrexate can cause bone-marrow suppression resulting in decreased production of all kinds of blood cells, particularly when used in combination with any of a number of other drugs including corticosteroids, non-steroidal anti-inflammatory drugs, cyclosporin, trimethoprim and certain antibiotics. Although methotrexate is generally well tolerated in the dosing regimens used for the treatment of arthritis (or psoriasis), even at these lower doses methotrexate can cause bone-marrow suppression and especially neutropenia. For example, in case reports (Sosin & Handa, Brit. Med. J. 326: 266-267, 2003) neutropenia was reported in patients treated with weekly doses of methotrexate of between 5 mg and 17.5 mg. Recommendations for methotrexate dosing in RA, such as the British Society for Rheumatology's guidelines (July 2000) are 7.5 mg Methotrexate weekly, increasing by 2.5 mg every six weeks to a maximum weekly dose of 25 mg. Thus neutropenia developed in these patients at doses significantly below the recommended maximum dose, especially with concomitant therapies.

In chemotherapy including methotrexate, GM-CSF may be prescribed to correct the low neutrophil levels in the blood and hence reduce the duration and severity of the neutropenia (Am. Soc. Clin. Onc. 2006, J. Clin. Oncol. 24: July 1st 2006). In this clinical setting, GM-CSF is used as a hematopoietic growth factor to enhance the production of granulocytes (including neutrophils) and macrophages. For example, short-term administration of GM-CSF to cancer patients can lead to a rapid increase in neutrophil counts and reduces neutropenia in patients treated with chemotherapy regime including methotrexate (Aglietta et al., Cancer 72: 2970-2973, 1993). The established efficacy of GM-CSF in treating neutropenia due to methotrexate raises concerns that antagonism of GM-CSF may have the opposite effect, *i.e.,* GM-CSF antagonism may contribute to neutropenia, particularly in patients concomitantly or previously treated with methotrexate.

Neutropenia is a significant and serious side-effect of several current therapies for inflammatory arthritis including cytokine antagonists. The IL-1 antagonist anakinra leads to an increased risk of neutropenia, both alone and particularly when used in combination with a TNF-antagonist (Fleischmann et al., Expert Opinion Biol Ther. 4:1333, 2004). Infliximab has also been associated with an increased risk of neutropenia.

here is currently a need for additional treatments of RA, particularly in patients receiving anti-folate compounds such as methotrexate. The current invention addresses this need.

### BRIEF SUMMARY OF THE INVENTION

The present invention provides methods to treat a patient suffering from a chronic inflammatory condition such as an inflammatory arthritic condition, *e.g.*, RA, with a GM-CSF antagonist. In typical embodiments, the GM-CSF antagonist is administered in combination with an anti-folate compound, *e.g.*, methotrexate, in amounts that do not cause neutropenia. In some embodiments, the GM-CSF antagonist is recombinantly produced, *e.g.*, a recombinant monoclonal antibody. In other embodiments, the GM-CSF antagonist, *e.g.*, purified anti-GM-CSF from human plasma, is purified from a natural source.

In one aspect, the invention provides a method for treating a patient suffering from a chronic inflammatory disease, *e.g.*, rheumatoid arthritis, the method comprising administering an anti-folate compounds, *e.g.*, methotrexate, and administering a GM-CSF antagonist to the patient, wherein the anti-folate compound, *e.g.*, methotrexate, and the GM-CSF antagonist are provided in an amount sufficient to reduce the symptoms of the chronic inflammatory disease, but in an amount that does not induce neutropenia. A GM-CSF antagonist can be *e.g.,* an anti-GM-CSF antibody, an anti-GM-CSF receptor antibody; a soluble GM-CSF receptor; a cytochrome b562 antibody mimetic; an adnectin, a lipocalin scaffold antibody mimetic; a calixarene antibody mimetic, or an antibody like binding peptidomimetic.

In many embodiments, the GM-CSF antagonist is an antibody to GM-CSF, *i.e.*, an anti-GM-CSF antibody. In various embodiments, the antibody can be a polyclonal antibody, a monoclonal antibody, or an antibody such as a nanobody or a camelid antibody. In some embodiments, the antibody is an antibody fragment, such as a Fab, a Fab', a F(ab')₂, a scFv, or a domain antibody (dAB). The antibody can also be modified, *e.g.*, to enhance stability. Thus, in some embodiments, the antibody is conjugated to polyethylene glycol.

In some embodiments, the antibody has an affinity of about 100 pM to about 10 nM, *e.g.*, from about 100 pM, about 200 pM, about 300 pM, about 400 pM, about 500 pM, about 600 pM, about 700 pM, about 800 pM, about 900 pM, or about 1 nM to about 10 nM. In further embodiments, the antibody has an affinity of about 1 pM to about 100 pM, *e.g.*, an affinity of about 1 pM, about 5 pM, about 10 pM, about 15 pM, about 20 pM, about 25 pM, about 30 pM, about 40 pM, about 50 pM, about 60 pM, about 70 pM, about 80 pM, or about 90 pM to about 100 pM. In some embodiments, the antibody has an affinity of from about 10 to about 30 pM.

In some embodiments, the antibody is a neutralizing antibody. In further embodiments, the antibody is a recombinant or chimeric antibody. In some embodiments, the antibody is a human antibody. In some embodiments, the antibody comprises a human variable region. In some embodiments, the antibody comprises a human light chain constant region. In some embodiments, the antibody comprises a human heavy chain constant region, such as a gamma chain.

In further embodiments, the antibody binds to the same epitope as a chimeric 19/2 antibody. The antibody can, e.g., comprise the V_{H} and V_{L} regions of chimeric 19/2. The antibody can also comprise a human heavy chain constant region such as a gamma region. In some embodiments, the antibody comprises the CDR1, CDR2, and CDR3 of the V_{H} region of chimeric 19/2. In further embodiments, the antibody comprises the CDR1, CDR2, and CDR3 of the V_{L} region of chimeric 19/2. In additional embodiments, the antibody comprises the CDR1, CDR2, and CDR3 of the V_{H} and V_{L} regions of a chimeric 19/2 antibody. In some embodiments, the antibody comprises the V_{H} region CDR3 and V_{L} region CDR3 of chimeric 19/2.

In some embodiments, the antibody has a half-life of about 7 to about 25 days.

In some embodiments of the methods of the invention, the GM-CSF antagonist, *e.g.*, an anti-GMCSF antibody, is administered by injection or by infusion. For example, the GM-CSF antagonist can be administered intravenously over a period between about 15 minutes and about 2 hours.

In other embodiments, the GM-CSF antagonist is administered subcutaneously by bolus injection.

In further embodiments, the GM-CSF antagonist is administered intramuscularly.

A GM-CSF antibody can, for example, be administered at a dose between about 1 mg/kg of body weight and about 10 mg/kg of body weight.

In some embodiments, treatment with the GM-CSF antagonist comprises a second administration of the GM-CSF antagonist.

The invention also provides a method of treating a chronic inflammatory disease, *e.g.*, rheumatoid arthritis, the method comprising administering an anti-GM-CSF antibody as described herein in a therapeutically effective amount. n some embodiments, the anti-GM-CSF antagonist, *e.g.*, an anti-GM-CSF antibody, is administered to a patient that has a neurodegenerative disease such as Alzheimer's disease.

### DETAILED DESCRIPTION OF THE INVENTION

### Definitions

As used herein, "chronic inflammatory disease" refers to diseases associated with an inflammatory response of prolonged duration. In some instances, the inflammatory response can last weeks, months or even indefinitely. The extended duration of the inflammatory response is frequently provoked by a persistent stimulus to the inflammatory response. The inflammatory response causes tissue damage. Chronic inflammation can be the result of progression of acute inflammation. Chronic inflammation can also ensue after repeat episodes of acute inflammation or can develop *de novo.* A number of inflammatory illnesses have been found to be associated with persistent pathogen infection, irritant non-living foreign matter that cannot be removed by enzymatic break-down or phagocytosis, or a "normal" tissue component that is recognized as non-self (most frequently associated with auto-immune diseases). The histological appearance of chronic inflammation frequently involves a mixed inflammatory cell infiltrate which is most often associated with the presence of macrophages, lymphocytes and plasma cells with neutrophil and eosinophil polymorphs as possible minor components (neutrophil and eosinophil polymorphs are associated in greater numbers with acute inflammation). Examples of inflammatory diseases include arthritis, *e.g.*, RA, psoriatic arthritis, ankylosing spondylitis, juvenile idiopathic arthritis, and other inflammatory diseases of the joint; inflammatory bowel diseases, *e.g.*, ulcerative colitis, Crohn's disease, Barrett's syndrome, ileitis, enteritis, and gluten-sensitive enteropathy; inflammatory disorders of the respiratory system, such as asthma, adult respiratory distress syndrome, allergic rhinitis, silicosis, chronic obstructive airway disease, hypersensitivity lung diseases, bronchiectasis; inflammatory diseases of the skin, including psoriasis, scleroderma, and inflammatory dermatoses such as eczema, atopic dermatitis, urticaria, and pruritis; disorders involving inflammation of the central and peripheral nervous system, including multiple sclerosis, idiopathic demyelinating polyneuropathy, Guillain-Barre syndrome, chronic inflammatory demyelinating polyneuropathy, and neurodegenerative diseases such as Alzheimer's disease. Various other inflammatory diseases can be treated using the methods of the invention. These include systemic lupus erythematosis, immune-mediated renal disease, *e.g.*, glomerulonephritis, and spondyloarthropathies; and diseases with an undesirable chronic inflammatory component such as systemic sclerosis, idiopathic inflammatory myopathies, Sjogren's syndrome, vasculitis, sarcoidosis, thyroiditis, gout, otitis, conjunctivitis, sinusitis, sarcoidosis, Behcet's syndrome, hepatobiliary diseases such as hepatitis, primary biliary cirrhosis, granulomatous hepatitis, and sclerosing cholangitis; inflammation and ischemic injury to the cardiovascular system such as ischemic heart disease, stroke, and atherosclerosis; and graft rejection, including allograft rejection and graft-v-host disease. Various other inflammatory diseases include tuberculosis and chronic cholecystitis. Additional chronic inflammatory diseases are described, *e.g.*, in Harrison's Principles of Internal Medicine, 12th Edition, Wilson, et al., eds., McGraw-Hill, Inc.).

The term "rheumatoid arthritis" (RA) refers to chronic inflammatory disease that develops as an auto-immune disorder and is associated with chronic inflammation of the joints. Frequently, the inflammation spreads to tissues surrounding the joints and to other organs. Typically,RA is a progressive illness that can cause join destruction and functional disability. The joint inflammation associated with RA causes swelling, pain, stiffness, and redness in the joints. The inflammation of rheumatoid disease can also occur in tissues around the joints, such as the tendons, ligaments, and muscles. In some patients with RA, chronic inflammation leads to the destruction of the cartilage, bone and ligaments causing deformity of the joints. Damage to the joints can occur early in the disease and be progressive. Progressive damage to the joints does not necessarily correlate with the degree of pain, stiffness, or swelling present in the joints.

As used herein, "Granulocyte Macrophage-Colony Stimulating Factor" (GM-CSF) refers to a small a naturally occurring glycoprotein with internal disulfide bonds having a molecular weight of approximately 23 kDa. In humans, it is encoded by a gene located within the cytokine cluster on human chromosome 5. The sequence of the human gene and protein are known. The protein has an N-terminal signal sequence, and a C-terminal receptor binding domain (Rasko and Gough In: The Cytokine Handbook, A. Thomson, et al, Academic Press, New York (1994) pages 349-369). Its three-dimensional structure is similar to that of the interleukins, although the amino acid sequences are not similar. GM-CSF is produced in response to a number of inflammatory mediators by mesenchymal cells present in the hemopoietic environment and at peripheral sites of inflammation. GM-CSF is able to stimulate the production of neutrophilic granulocytes, macrophages, and mixed granulocyte-macrophage colonies from bone marrow cells and can stimulate the formation of eosinophil colonies from fetal liver progenitor cells. GM-CSF can also stimulate some functional activities in mature granulocytes and macrophages.

The term "granulocyte macrophage-colony stimulating factor receptor" (GM-CSFR)" refers to a membrane bound receptor expressed on cells that transduces a signal when bound to granulocyte macrophage colony-stimulating factor (GM-CSF). GM-CSFR consists of a ligand-specific low-affinity binding chain (GM-CSFR alpha) and a second chain that is required for high-affinity binding and signal transduction. This second chain is shared by the ligand-specific alpha-chains for the interleukin 3 (IL-3) and IL-5 receptors and is therefore called beta common (beta c). The cytoplasmic region of GM-CSFR alpha consists of a membrane-proximal conserved region shared by the alpha 1 and alpha 2 isoforms and a C-terminal variable region that is divergent between alpha 1 and alpha 2. The cytoplasmic region of beta-c contains membrane proximal serine and acidic domains that are important for the proliferative response induced by GM-CSF

The term "soluble granulocyte macrophage-colony stimulating factor receptor" (sGM-CSFR) refers to a non-membrane bound receptor that binds GM-CSF, but does not transduce a signal when bound to the ligand.

As used herein, a "peptide GM-CSF antagonist" refers to a peptide that interacts with GM-CSF, or its receptor, to reduce or block (either partially or completely) signal transduction that would otherwise result from the binding of GM-CSF to its cognate receptor expressed on cells. GM-CSF antagonists may act by reducing the amount of GM-CSF ligand available to bind the receptor (*e.g.*, antibodies that once bound to GM-CSF increase the clearance rate of GM-CSF) or prevent the ligand from binding to its receptor either by binding to GM-CSF or the receptor (*e.g.*, neutralizing antibodies). GM-CSF antagonist may also include other peptide inhibitors, which may include polypeptides that bind GM-CSF or its receptor to partially or completely inhibit signaling. A peptide GM-CSF antagonist can be, *e.g.*, an antibody; a natural or synthetic GM-CSF receptor ligand that antagonizes GM-CSF, or other polypeptides. An exemplary assay to detect GM-CSF antagonist activity is provided in Example 1. Typically, peptide GM-CSF antagonist, such as a neutralizing antibody, has an EC₅₀ of 10 nM or less.

A "purified" GM-CSF antagonist as used herein refers to a GM-CSF antagonist that is substantially or essentially free from components that normally accompany it as found in its native state. For example, a GM-CSF antagonist such as an anti-GM-CSF antibody, that is purified from blood or plasma is substantially free of other blood or plasma components such as other immunoglobulin molecules. Purity and homogeneity are typically determined using analytical chemistry techniques such as polyacrylamide gel electrophoresis or high performance liquid chromatography. A protein that is the predominant species present in a preparation is substantially purified. Typically, "purified" means that the protein is at least 85% pure, more preferably at least 95% pure, and most preferably at least 99% pure relative to the components with which the protein naturally occurs..

As used herein, an "antibody" refers to a protein functionally defined as a binding protein and structurally defined as comprising an amino acid sequence that is recognized by one of skill as being derived from the framework region of an immunoglobulin-encoding gene of an animal that produces antibodies. An antibody can consist of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

A typical immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains, respectively.

The term "antibody" as used herein includes antibody fragments that retain binding specificity. For example, there are a number of well characterized antibody fragments. Thus, for example, pepsin digests an antibody below the disulfide linkages in the hinge region to produce F(ab)'₂, a dimer of Fab which itself is a light chain joined to VH-CH1 by a disulfide bond. The F(ab)'₂ may be reduced under mild conditions to break the disulfide linkage in the hinge region thereby converting the (Fab')₂ dimer into an Fab' monomer. The Fab' monomer is essentially a Fab with part of the hinge region (see, Fundamental Immunology, W.E. Paul, ed., Raven Press, N.Y. (1993), for a more detailed description of other antibody fragments). While various antibody fragments are defined in terms of the digestion of an intact antibody, one of skill will appreciate that fragments can be synthesized de novo either chemically or by utilizing recombinant DNA methodology. Thus, the term antibody, as used herein also includes antibody fragments either produced by the modification of whole antibodies or synthesized using recombinant DNA methodologies.

Antibodies include dimers such as V_{H}-V_{L} dimers, V_{H} dimers, or V_{L} dimers, including single chain antibodies (antibodies that exist as a single polypeptide chain), such as single chain Fv antibodies (sFv or scFv) in which a variable heavy and a variable light region are joined together (directly or through a peptide linker) to form a continuous polypeptide. The single chain Fv antibody is a covalently linked V_{H}-V_{L} heterodimer which may be expressed from a nucleic acid including V_{H}- and V_{L}- encoding sequences either joined directly or joined by a peptide-encoding linker (*e.g.*, Huston, et al. Proc. Nat. Acad. Sci. USA, 85:5879-5883, 1988). While the V_{H} and V_{L} are connected to each as a single polypeptide chain, the V_{H} and V_{L} domains associate non-covalently. Alternatively, the antibody can be another fragment, such as a disulfide-stabilized Fv (dsFv). Other fragments can also be generated, including using recombinant techniques. The scFv antibodies and a number of other structures converting the naturally aggregated, but chemically separated light and heavy polypeptide chains from an antibody V region into a molecule that folds into a three dimensional structure substantially similar to the structure of an antigen-binding site are known to those of skill in the art (see e.g., U.S. Patent Nos. 5,091,513, 5,132,405, and 4,956,778). In some embodiments, antibodies include those that have been displayed on phage or generated by recombinant technology using vectors where the chains are secreted as soluble proteins, *e.g.*, scFv, Fv, Fab, (Fab')₂ or generated by recombinant technology using vectors where the chains are secreted as soluble proteins. Antibodies for use in the invention can also include diantibodies and miniantibodies.

Antibodies of the invention also include heavy chain dimers, such as antibodies from camelids. Since the V_{H} region of a heavy chain dimer IgG in a camelid does not have to make hydrophobic interactions with a light chain, the region in the heavy chain that normally contacts a light chain is changed to hydrophilic amino acid residues in a camelid. V_{H} domains of heavy-chain dimer IgGs are called VHH domains. Antibodies for use in the current invention include single domain antibodies (dAbs) and nanobodies (*see*, *e.g.*, Cortez-Retamozo, et al., Cancer Res. 64:2853-2857, 2004).

As used herein, "V-region" refers to an antibody variable region domain comprising the segments of Framework 1, CDR1, Framework 2, CDR2, and Framework 3, including CDR3 and Framework 4, which segments are added to the V-segment as a consequence of rearrangement of the heavy chain and light chain V-region genes during B-cell differentiation. A "V-segment" as used herein refers to the region of the V-region (heavy or light chain) that is encoded by a V gene.

As used herein, "complementarity-determining region (CDR)" refers to the three hypervariable regions in each chain that interrupt the four "framework" regions established by the light and heavy chain variable regions. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, for example, a V_{H} CDR3 is located in the variable domain of the heavy chain of the antibody in which it is found, whereas a V_{L} CDR1 is the CDR1 from the variable domain of the light chain of the antibody in which it is found.

The sequences of the framework regions of different light or heavy chains are relatively conserved within a species. The framework region of an antibody, that is the combined framework regions of the constituent light and heavy chains, serves to position and align the CDRs in three dimensional space.

The amino acid sequences of the CDRs and framework regions can be determined using various well known definitions in the art, *e.g.*, Kabat, Chothia, international ImMunoGeneTics database (IMGT), and AbM (*see, e.g.*, Johnson *et al.*, *supra*; Chothia & Lesk, 1987, Canonical structures for the hypervariable regions of immunoglobulins. J. Mol. Biol. 196, 901-917; Chothia C. et al., 1989, Conformations of immunoglobulin hypervariable regions. Nature 342, 877-883; Chothia C. et al., 1992, structural repertoire of the human VH segments J. Mol. Biol. 227, 799-817; Al-Lazikani et al., J.Mol.Biol 1997, 273(4)). Definitions of antigen combining sites are also described in the following: Ruiz et al., IMGT, the international ImMunoGeneTics database. Nucleic Acids Res., 28, 219-221 (2000); and Lefranc,M.-P. IMGT, the international ImMunoGeneTics database. Nucleic Acids Res. Jan 1;29(1):207-9 (2001); MacCallum et al, Antibody-antigen interactions: Contact analysis and binding site topography, J. Mol. Biol., 262 (5), 732-745 (1996); and Martin et al, Proc. Natl Acad. Sci. USA, 86, 9268-9272 (1989); Martin, et al, Methods Enzymol., 203, 121-153, (1991); Pedersen et al, Immunomethods, 1, 126, (1992); and Rees et al, In Sternberg M.J.E. (ed.), Protein Structure Prediction. Oxford University Press, Oxford, 141-172 1996).

"Epitope" or "antigenic determinant" refers to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, e.g., Epitope Mapping Protocols in Methods in Molecular Biology, Vol. 66, Glenn E. Morris, Ed (1996).

As used herein, "neutralizing antibody" refers to an antibody that binds to GM-CSF and prevents signaling by the GM-CSF receptor, or inhibits binding of GM-CSF to its receptor.

As used herein, "chimeric antibody" refers to an immunoglobulin molecule in which (a) the constant region, or a portion thereof, is altered, replaced or exchanged so that the antigen binding site (variable region) is linked to a constant region of a different or altered class, effector function and/or species, or an entirely different molecule that confers new properties to the chimeric antibody, e.g., an enzyme, toxin, hormone, growth factor, drug, etc.; or (b) the variable region, or a portion thereof, is altered, replaced or exchanged with a variable region, or portion thereof, having a different or altered antigen specificity; or with corresponding sequences from another species or from another antibody class or subclass.

As used herein, "humanized antibody" refers to an immunoglobulin molecule in which the CDRs of a recipient human antibody are replaced by CDRs from a donor antibody. Humanized antibodies may also comprise residues of donor origin in the framework sequences. The humanized antibody can also comprise at least a portion of a human immunoglobulin constant region.. Humanized antibodies may also comprise residues which are found neither in the recipient antibody nor in the imported CDR or framework sequences. Humanization can be performed using methods known in the art (*e.g.*, Jones et al., Nature 321:522-525; 1986; Riechmann et al., Nature 332:323-327, 1988; Verhoeyen et al., Science 239:1534-1536, 1988); Presta, Curr. Op. Struct. Biol. 2:593-596, 1992; U.S. Patent No. 4,816,567), including techniques such as "superhumanizing" antibodies (Tan et al., J. Immunol. 169: 1119, 2002) and "resurfacing" (e.g., Staelens et al., Mol. Immunol. 43: 1243, 2006; and Roguska et al., Proc. Natl. Acad. Sci USA 91: 969, 1994).

A "humaneered" antibody in the context of this invention refers to an engineered human antibody having a binding specificity of a reference antibody. A "humaneered" antibody for use in this invention has an immunoglobulin molecule that contains minimal sequence derived from non-human immunoglobulin. Typically, an antibody is "humaneered" by joining a DNA sequence encoding a binding specificity determinant (BSD) from the CDR3 region of the heavy chain of the reference antibody to human V_{H} segment sequence and a light chain CDR3 BSD from the reference antibody to a human V_{L} segment sequence. A "BSD" refers to a CDR3-FR4 region, or a portion of this region that mediates binding specificity. A binding specificity determinant therefore can be a CDR3-FR4, a CDR3, a minimal essential binding specificity determinant of a CDR3 (which refers to any region smaller than the CDR3 that confers binding specificity when present in the V region of an antibody), the D segment (with regard to a heavy chain region), or other regions of CDR3-FR4 that confer the binding specificity of a reference antibody. Methods for humaneering are provided in US patent application publication no. 20050255552 and US patent application publication no. 20060134098.

The term "heterologous" when used with reference to portions of a nucleic acid indicates that the nucleic acid comprises two or more subsequences that are not normally found in the same relationship to each other in nature. For instance, the nucleic acid is typically recombinantly produced, having two or more sequences, e.g., from unrelated genes arranged to make a new functional nucleic acid. Similarly, a heterologous protein will often refer to two or more subsequences that are not found in the same relationship to each other in nature.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, e.g., recombinant cells express genes that are not found within the native (non-recombinant) form of the cell or express native genes that are otherwise abnormally expressed, under expressed or not expressed at all. By the term "recombinant nucleic acid" herein is meant nucleic acid, originally formed in vitro, in general, by the manipulation of nucleic acid, e.g., using polymerases and endonucleases, in a form not normally found in nature. In this manner, operably linkage of different sequences is achieved. Thus an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, i.e., using the in vivo cellular machinery of the host cell rather than in vitro manipulations; however, such nucleic acids, once produced recombinantly, although subsequently replicated non-recombinantly, are still considered recombinant for the purposes of the invention. Similarly, a "recombinant protein" is a protein made using recombinant techniques, i.e., through the expression of a recombinant nucleic acid as depicted above.

The phrase "specifically (or selectively) binds" to an antibody or "specifically (or selectively) immunoreactive with," when referring to a protein or peptide, refers to a binding reaction that is determinative of the presence of the protein, in a heterogeneous population of proteins and other biologics. Thus, under designated immunoassay conditions, the specified antibodies bind to a particular protein sequence at least two times the background and more typically more than 10 to 100 times background.

Specific binding to an antibody under such conditions requires an antibody that is selected for its specificity for a particular protein. For example, polyclonal antibodies raised to a particular protein, polymorphic variants, alleles, orthologs, and conservatively modified variants, or splice variants, or portions thereof, can be selected to obtain only those polyclonal antibodies that are specifically immunoreactive with GM-CSF protein and not with other proteins. This selection may be achieved by subtracting out antibodies that cross-react with other molecules.

As used herein, a "therapeutic agent for a chronic inflammatory disease" refers to an agent that when administered to a patient suffering from a chronic inflammatory disease, in a therapeutically effective dose, will cure, or at least partially arrest the symptoms of the disease and complications associated with the disease.

The terms "identical" or percent "identity," in the context of two or more polypeptide (or nucleic acid) sequences, refer to two or more sequences or subsequences that are the same or have a specified percentage of amino acid residues (or nucleotides) that are the same (i.e., about 60% identity, preferably 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (*see, e.g.*, NCBI web site). Such sequences are then said to be "substantially identical." "Substantially identical" sequences also includes sequences that have deletions and/or additions, as well as those that have substitutions, as well as naturally occurring, *e.g.*, polymorphic or allelic variants, and man-made variants. As described below, the preferred algorithms can account for gaps and the like. Preferably, protein sequence identity exists over a region that is at least about 25 amino acids in length, or more preferably over a region that is 50-100 amino acids = in length, or over the length of a protein.

A "comparison window", as used herein, includes reference to a segment of one of the number of contiguous positions selected from the group consisting typically of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, *e.g.*, by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Current Protocols in Molecular Biology (Ausubel et al., eds. 1995 supplement)).

Preferred examples of algorithms that are suitable for determining percent sequence identity and sequence similarity include the BLAST and BLAST 2.0 algorithms, which are described in Altschul et al., Nuc. Acids Res. 25:3389-3402 (1977) and Altschul et al., J. Mol. Biol. 215:403-410 (1990). BLAST and BLAST 2.0 are used, with the parameters described herein, to determine percent sequence identity for the nucleic acids and proteins of the invention. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands.

An indication that two polypeptides are substantially identical is that the first polypeptide is immunologically cross reactive with the antibodies raised against the second polypeptide. Thus, a polypeptide is typically substantially identical to a second polypeptide, e.g., where the two peptides differ only by conservative substitutions.

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. A protein that is the predominant species present in a preparation is substantially purified. The term "purified" in some embodiments denotes that a protein gives rise to essentially one band in an electrophoretic gel. Preferably, it means that the protein is at least 85% pure, more preferably at least 95% pure, and most preferably at least 99% pure.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, e.g., an α carbon that is bound to a hydrogen, a carboxyl group, an amino group, and an R group, e.g., homoserine, norleucine, methionine sulfoxide, methionine methyl sulfonium. Such analogs may have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical or associated, e.g., naturally contiguous, sequences. Because of the degeneracy of the genetic code, a large number of functionally identical nucleic acids encode most proteins. For instance, the codons GCA, GCC, GCG and GCU all encode the amino acid alanine. Thus, at every position where an alanine is specified by a codon, the codon can be altered to another of the corresponding codons described without altering the encoded polypeptide. Such nucleic acid variations are "silent variations," which are one species of conservatively modified variations. Every nucleic acid sequence herein which encodes a polypeptide also describes silent variations of the nucleic acid. One of skill will recognize that in certain contexts each codon in a nucleic acid (except AUG, which is ordinarily the only codon for methionine, and TGG, which is ordinarily the only codon for tryptophan) can be modified to yield a functionally identical molecule. Accordingly, often silent variations of a nucleic acid which encodes a polypeptide is implicit in a described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables and substitution matrices such as BLOSUM providing functionally similar amino acids are well known in the art. Such conservatively modified variants are in addition to and do not exclude polymorphic variants, interspecies homologs, and alleles of the invention. Typical conservative substitutions for one another include: 1) Alanine (A), Glycine (G); 2) Aspartic acid (D), Glutamic acid (E); 3) Asparagine (N), Glutamine (Q); 4) Arginine (R), Lysine (K); 5) Isoleucine (1), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

### I. INTRODUCTION

The invention relates to methods of administering a GM-CSF antagonist for the treatment of patients diagnosed with a chronic inflammatory disease. In some embodiments, the patient is undergoing treatment with an anti-folate compound such as methotrexate. In some embodiments, chronic inflammatory diseases that are treated with a GM-CSF antagonist, e.g., an anti-GM-CSF antibody, include inflammatory arthritis diseases, such as RA, psoriatic arthritis, ankylosing spondylitis, and juvenile idiopathic arthritic; as well as other inflammatory diseases such as polymyositis, and systemic lupus erythermatosus. Patients having such disorders may, in some embodiments, also be undergoing treatment with an anti-folate compound such as methotrexate. In embodiments where the GM-CSF antagonist is administered with an anti-folate compound such as methotrexate, the GM-CSF and anti-folate compounds, e.g., methotrexate, are administered in an amount that does not induce neutropenia.. GM-CSF antagonists may include anti-GM-CSF antibodies, anti-GM-CSF receptor antibodies, or other inhibitors that prevent signaling that normally results from the binding of GM-CSF to its cognate receptor.

In some embodiments, the invention provides a method of treating a chronic inflammatory disease, e.g., rheumatoid arthritis, by administering an anti-GMCSF antibody as described herein. Further chronic inflammatory diseases that can be treated with a GM-CSF antagonist, e.g., an anti-GMCSF antibody, include neurodegenerative diseases, such as Alzheimer's disease.

Antibodies, e.g., anti-GM-CSF or anti-GM-CSF receptor antibodies, suitable for use with the present invention may be monoclonal, polyclonal, chimeric, humanized, humaneered, or human. Other GM-CSF antagonists suitable for use with the present invention may include naturally occurring or synthetic ligands (or fragments thereof) that compete with GM-CSF for binding to the receptor, but do not result in signaling when bound to the receptor. Additional non-limiting GM-CSF antagonists may include polypeptides, nucleic acids, small molecules and the like that either partially or completely block signaling that would naturally result from the binding of GM-CSF to its receptor in the absence of the GM-CSF antagonist.

### II. Patients

Typical patients to be treated with the GM-CSF antagonist are those having a chronic inflammatory disorder who are also undergoing treatment with methotrexate who have not developed neutropenia. Patients are treated with methotrexate according to established clinical guidelines and are treated with weekly doses of methotrexate in the range of about 5 to about 25 mg of methotrexate per week. For some patients, lower doses of methotrexate might be suitable and can include a weekly regimen of between about 0.1 and about 5 mg of methotrexate. In other embodiments, the amount of methotrexate administered is between about 5 mg/week and about 25 mg/week.

In some embodiments, a patient that is treated with a GM-CSF antagonist, such as an anti-GM-CSF antibody, is undergoing treatment with an analog of methotrexate or another anti-folate therapeutic agent. Methotrexate is structurally similar to folate and can bind to the active sites of a number of enzymes that normally use folate as a coenzyme for the biosynthesis of the purine and pyrimidine nucleotide precursors of DNA and for the interconversion of amino acids during protein biosynthesis. Methotrexate competes with the folate cofactor for enzyme binding sites, thereby inhibiting enzyme activity. A "methotrexate analog" is a compound having structural similarity to methotrexate that also has anti-folate activity. Thus, methotrexate analogs also refers to derivatives, and prodrugs that may be used in the practice of this invention. For example, prodrugs may be used to increase bioavailability through selective bioconversion. Methotrexate anlaogs include, *e.g.*, 4-amino derivatives with halogen substitution on the para-aminobenzoic moiety, such as dichloromethotrexate (see, *e.g.*, Frei et al., Clin. Pharmacol. Therap., 6:160-71 (1965)); 7-methyl substituted methotrexate (see, *e.g.*, Rosowsky et al., J. Med. Chem., 17:1308-11 (1974)); 3',5'-difluoro methotrexate (see, *e.g.*, Tomcuf, J. Organic Chem., 26:3351 (1961)); 2' and 3' monofluorinated derivatives of aminopterin (see, *e.g.*, Henkin et al., J. Med. Chem., 26:1193-1196 (1983)); and 7,8-dihydro-8-methyl-methotrexate (see, *e.g.,* Chaykovsky, J. Org. Chem., 40:145-146 (1975)).

As used herein, the term "anti-folate compound" refers to a compound having structural similarity to folate and activity as a folate antagonist against one or more folate-dependent enzymes. Examples of anti-folate compounds include, e.g., aminopterin, raltitrexed, lometrexol, multitargeted anti-folate (MTA), AQA, methotrexate, and analogs thereof. Aminopterin, for example, possesses a hydrogen instead of a methyl group at position N-10 compared to the structure of methotrexate. Raltitrexed (ZD1694) is a selective inhibitor of thymidylate synthase. Lometrexol selectively inhibits glycinamide ribonucleotide formyltransferase, the first enzyme involved in the pathway of de novo purine synthesis. Other anti-folate compounds include, for example, trimetrexate, edetrexate, and the like (see, *e.g.*, Takimoto, Oncologist 1:68-81, 1996, for a listing of exemplary anti-folate compounds). In certain instances, methotrexate can be used in a combination therapy with one or more methotrexate analogs and/or other anti-folate compounds and an anti-GMCSF antagonist. The anti-folate agents are administered in an amount that does not produce neutropenia. In some embodiments, the amount is from about 0.1, *e.g.*, about 0.5, about 1, about 2, about 3, about 4, about 5, about 7.5, about 10, about 12.5, about 15, about 20 to about 25 mg per week. The amount of anti-folate compound administered as an anti-inflammatory agent is often an amount that is two to three log orders lower than amounts of anti-folate compounds used in treating cancer.

In some embodiments, a patient suffering from an inflammatory arthritis is treated according to the methods of the present invention. Such patients include those suffering from RA, psoriatic arthritis, ankylosing spondylitis, or juvenile idiopatic arthritis.

Methods well known in the art can be used to determine if a patient is neutropenic. The absolute neutrophil count (ANC) is used to determine if the patient is neutropenic. Patients are not considered neutropenic if the neutrophil and white blood cell counts (WBCC) are within the normal range. The normal range for neutrophils is understood to be represented by a count of greater than 1x10⁹/l; the normal range for white blood cells is understood to be represented by a count greater than 3.5x10⁹/l. Neutropenia is considered clinically significant if the ANC is less than 0.5x10⁹/l. In some embodiments, no clinically significant neutropenia is induced in patients treated according to the methods of the present invention. In some other embodiments, the treatment causes no detectable neutropenia.

In some embodiments, a patient that has active RA is treated in accordance with the methods of the invention. The response of a RA patient to a therapy and/or disease progression can be evaluated by monitoring any of the clinical parameters associated with RA. Typically, a patient that exhibits a therapeutic response to treatment is determined by a number of parameters, including pharmacological parameters. For example, American College for Rheumatology (ACR) scoring for RA (ACR 20, ACR 50 and ACR 70) can be employed. ACR composite end-points for RA include: morning stiffness, tender joint count, swollen joint count, patient pain assessment, patient global assessment, physician global assessment; erythrocyte sedimentation rate (ESR), C-reactive protein (CRP) levels in plasma, and measure of rheumatoid factor. Other pharmacodynamic markers that can be used to evaluate patient response include evaluation of neopterin levels in blood or in urine, evaluation of levels of pro-inflammatory cytokines systemically (in the blood) or locally (e.g., at a localized site of inflammation such as a joint). Pro inflammatory cytokines are well known in the art. Examples of pro-inflammatory cytokines that can be used to evaluate patient response to the therapy of this invention include, but are not limited to, TNF-α, GM-CSF, Interleukin-1, Interleukin-6, and Interleukins-8 and -17.

Administration of GM-CSF antagonists with an anti-folate compound such as methotrexate at least partially arrests disease progression or reduces symptoms of the disease symptoms (as assessed by parameters such as the exemplary parameters noted above). Thus, administration of a GM-CSF antagonist and methotrexat can reduce the progression of joint erosion. Progression of joint erosion can be assessed using known techniques such as autoradiography to evaluate bone and cartilage in joints.

In other embodiments, a GM-CSF antagonist is administered to a patient that has another inflammatory arthritis, such as psoriatic arthritis, juvenile idiopathic arthritis, or ankylosing spondylitis, that is being treated with an anti-folate compound such as methotrexate. Such patients can be evaluated for response to a therapy and/or disease progression using known methods such as those used to evaluate RA or other appropriate disease scoring criteria. For example, for ankylosing spondylitis, the Assessment on Ankylosing Spondylitis Response Criteria (ASAS 20) may be used. (ASAS is a composite measure of improvement in AS symptoms that include total back pain, patient assessment of disease activity, inflammation and physical function). Similary, for evaluation of psoriatic arthritis, the Psoriatic Arthritis Response Criteria (PsARC) index may be used.

In further embodiments of the invention, patients with systemic lupus erythematosus who are receiving an anti-folate compound such as methotrexate for treatment are also treated with a GM-CSF antagonist. Response to therapy and/or disease progression can be measured, for example, using established criteria (*e.g.*, Hochberg, Arthritis Rheum 40:1725, 1997; Tan, et al., Arthritis Rheum 25:1271-7, 1982) to provide evaluate the Systemic Lupus Erythematosus Disease Activity Index (SLEDAI) of a patient being treated with the methods of the invention.

In some embodiments, a patient suffering from a chronic inflammatory disease such as rheumatoid arthritis, psoriatic arthritis, juvenile idiopathic arthritis, ankylosing spondylitis systemic lupus erythematosus, or a neurodegenerative disease such as Alzheimer's is treated with a GM-CSF antagonist without also receiving anti-folate therapy. Often, the GM-CSF antagonist administered to such patients is an anti-GM-CSF antibody.

### III. GM-CSF antagonists

As noted above, the invention provides methods for treating a chronic inflammatory disease, *e.g.*, RA, by administering a GM-CSF antagonist and methotrexate to a patient suffering from the disease. GM-CSF antagonists suitable for use in the invention selectively interfere with the induction of signaling by the GM-CSF receptor by causing a reduction in the binding of GM-CSF to the receptor. Such antagonists may include antibodies that bind the GM-CSF receptor, antibodies that bind GM-CSF, and other proteins or small molecules that compete for binding of GM-CSF to its receptor or inhibit signaling that normally results from the binding of the ligand to the receptor.

In many embodiments, the GM-CSF antagonist used in the invention is a protein, *e.g..*, an anti-GM-CSF antibody, an anti-GM-CSF receptor antibody, a soluble GM-CSF receptor, or a modified GM-CSF polypeptide that competes for binding with GM-CSF to a receptor, but is inactive. Such proteins are often produced using recombinant expression technology. Such methods are widely are widely known in the art. General molecular biology methods, including expression methods, can be found, *e.g.*, in instruction manuals, such as, Sambrook and Russell (2001) Molecular Cloning: A laboratory manual 3rd ed. Cold Spring Harbor Laboratory Press; Current Protocols in Molecular Biology (2006) John Wiley and Sons ISBN: 0-471-50338-X.

A variety of prokaryotic and/or eukaryotic based protein expression systems may be employed to produce a GM-CSF antagonist protein. Many such systems are widely available from commercial suppliers. These include both prokaryotic and eukaryotic expression systems.

### GM-CSF Antibodies

In some embodiments, the GM-CSF antagonist is an antibody that binds GM-CSF or an antibody that binds to the GM-CSF receptor α or β subunit. The antibodies can be raised against GM-CSF (or GM-CSF receptor) proteins, or fragments, or produced recombinantly. Antibodies to GM-CSF for use in the invention can be neutralizing or can be non-neutralizing antibodies that bind GM-CSF and increase the rate of *in vivo* clearance of GM-CSF such that the GM-CSF level in the circulation is reduced. Often, the GM-CSF antibody is a neutralizing antibody.

Methods of preparing polyclonal antibodies are known to the skilled artisan (e.g., Harlow & Lane, Antibodies, A Laboratory manual (1988); Methods in Immunology). Polyclonal antibodies can be raised in a mammal by one or more injections of an immunizing agent and, if desired, an adjuvant. The immunizing agent includes a GM-CSF or GM-CSF receptor protein, *e.g.*, a human GM-CSF or GM-CSF receptor protein, or fragment thereof.

In some embodiment, a GM-CSF antibody for use in the invention is purified from human plasma. In such embodiments, the GM-CSF antibody is typically a polyclonal antibody that is isolated from other antibodies present in human plasma. Such an isolation procedure can be performed, *e.g.*, using known techniques, such as affinity chromatography.

In some embodiments, the GM-CSF antagonist is a monoclonal antibody. Monoclonal antibodies may be prepared using hybridoma methods, such as those described by Kohler & Milstein, Nature 256:495 (1975). In a hybridoma method, a mouse, hamster, or other appropriate host animal, is typically immunized with an immunizing agent, such as human GM-CSF, to elicit lymphocytes that produce or are capable of producing antibodies that will specifically bind to the immunizing agent. Alternatively, the lymphocytes may be immunized *in vitro.* The immunizing agent preferably includes human GM-CSF protein, fragments thereof, or fusion protein thereof.

Human monoclonal antibodies can be produced using various techniques known in the art, including phage display libraries (Hoogenboom & Winter, J. Mol. Biol. 227:381 (1991); Marks et al., J. Mol. Biol. 222:581 (1991)). The techniques of Cole *et al.* and Boerner *et al.* are also available for the preparation of human monoclonal antibodies (Cole et al., Monoclonal Antibodies and Cancer Therapy, p. 77 (1985) and Boerner et al., J. Immunol. 147(1):86-95 (1991)). Similarly, human antibodies can be made by introducing of human immunoglobulin loci into transgenic animals, *e.g.*, mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, *e.g.*, in U.S. Patent Nos. 5,545,807; 5,545,806; 5,569,825; 5,625,126; 5,633,425; 5,661,016, and in the following scientific publications: Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); Lonberg & Huszar, Intern. Rev. Immunol. 13:65-93 (1995).

In some embodiments the anti-GM-CSF antibodies are chimeric or humanized monoclonal antibodies. As noted *supra*, humanized forms of antibodies are chimeric immunoglobulins in which residues from a complementary determining region (CDR) of human antibody are replaced by residues from a CDR of a non-human species such as mouse, rat or rabbit having the desired specificity, affinity and capacity.

An antibody that is employed in the invention can be in any format. For example, in some embodiments, the antibody can be a complete antibody including a constant region, *e.g.,* a human constant region, or can be a fragment or derivative of a complete antibody, *e.g.*, an Fd, a Fab, Fab', F(ab')₂, a scFv, an Fv fragment, or a single domain antibody, such as a nanobody or a camelid antibody. Such antibodies may additionally be recombinantly engineered by methods well known to persons of skill in the art. As noted above, such antibodies can be produced using known techniques.

In some embodiments of the invention, the antibody is additionally engineered to reduced immunogenicity, *e.g.*, so that the antibody is suitable for repeat administration. Methods for generating antibodies with reduced immunogenicity include humanization/humaneering procedures and modification techniques such as de-immunization, in which an antibody is further engineered, *e.g.*, in one or more framework regions, to remove T cell epitopes.

In some embodiments, the antibody is a humaneered antibody. A humaneered antibody is an engineered human antibody having a binding specificity of a reference antibody, obtained by joining a DNA sequence encoding a binding specificity determinant (BSD) from the CDR3 region of the heavy chain of the reference antibody to human VH segment sequence and a light chain CDR3 BSD from the reference antibody to a human VL segment sequence. Methods for humaneering are provided in US patent application publication no. 20050255552 and US patent application publication no. 20060134098.

An antibody can further be de-immunized to remove one or more predicted T-cell epitopes from the V-region of an antibody. Such procedures are described, for example, in WO 00/34317.

In some embodiments, the variable region is comprised of human V-gene sequences. For example, a variable region sequence can have at least 80% identity, or at least 85% identity, at least 90% identity, at least 95% identity, at least 96% identity, at least 97% identity, at least 98% identity, or at least 99% identity, or greater, with a human germline V-gene sequence.

An antibody used in the invention can include a human constant region. The constant region of the light chain may be a human kappa or lambda constant region. The heavy chain constant region is often a gamma chain constant region, for example, a gamma-1, gamma-2, gamma-3, or gamma-4 constant region.

In some embodiments, *e.g.*, where the antibody is a fragment, the antibody can be conjugated to another molecule, *e.g.*, to provide an extended half-life *in vivo* such as a polyethylene glycol (pegylation) or serum albumin. Examples of PEGylation of antibody fragments are provided in Knight et al (2004) Platelets 15: 409 (for abciximab); Pedley et al (1994) Br. J. Cancer 70: 1126 (for an anti-CEA antibody) Chapman et al (1999) Nature Biotech. 17 : 780.

### Antibody Specificity

An antibody for use in the invention binds to GM-CSF or GM-CSF receptor. Any number of techniques can be used to determine antibody binding specificity. *See, e.g.,* Harlow & Lane, Antibodies, A Laboratory Manual (1988) for a description of immunoassay formats and conditions that can be used to determine specific immunoreactivity of an antibody.

An exemplary antibody suitable for use with the present invention is c19/2. In some embodiments, a monoclonal antibody that competes for binding to the same epitope as c19/2, or that binds the same epitope as c19/2, is used. The ability of a particular antibody to recognize the same epitope as another antibody is typically determined by the ability of the first antibody to competitively inhibit binding of the second antibody to the antigen. Any of a number of competitive binding assays can be used to measure competition between two antibodies to the same antigen. For example, a sandwich ELISA assay can be used for this purpose. This is carried out by using a capture antibody to coat the surface of a well. A subsaturating concentration of tagged-antigen is then added to the capture surface. This protein will be bound to the antibody through a specific antibody: epitope interaction. After washing a second antibody, which has been covalently linked to a detectable moiety (e.g., HRP, with the labeled antibody being defined as the detection antibody) is added to the ELISA. If this antibody recognizes the same epitope as the capture antibody it will be unable to bind to the target protein as that particular epitope will no longer be available for binding. If however this second antibody recognizes a different epitope on the target protein it will be able to bind and this binding can be detected by quantifying the level of activity (and hence antibody bound) using a relevant substrate. The background is defined by using a single antibody as both capture and detection antibody, whereas the maximal signal can be established by capturing with an antigen specific antibody and detecting with an antibody to the tag on the antigen. By using the background and maximal signals as references, antibodies can be assessed in a pair-wise manner to determine epitope specificity.

A first antibody is considered to competitively inhibit binding of a second antibody, if binding of the second antibody to the antigen is reduced by at least 30%, usually at least about 40%, 50%, 60% or 75%, and often by at least about 90%, in the presence of the first antibody using any of the assays described above.

### Epitope Mapping

In some embodiments of the invention, an antibody is employed that binds to the same epitope as a known antibody, *e.g.*, c19/2. Method of mapping epitopes are well known in the art. For example, one approach to the localization of functionally active regions of human granulocyte-macrophage colony-stimulating factor (hGM-CSF) is to map the epitopes recognized by neutralizing anti-hGM-CSF monoclonal antibodies. For example, the epitope to which c19/2 (which has the same variable regions as the neutralizing antibody LMM102) binds has been defined using proteolytic fragments obtained by enzymic digestion of bacterially synthesized hGM-CSF (Dempsey, et al., Hybridoma 9:545-558, 1990). RP-HPLC fractionation of a tryptic digest resulted in the identification of an immunoreactive "tryptic core" peptide containing 66 amino acids (52% of the protein). Further digestion of this "tryptic core" with S. aureus V8 protease produced a unique immunoreactive hGM-CSF product comprising two peptides, residues 86-93 and 112-127, linked by a disulfide bond between residues 88 and 121. The individual peptides, were not recognized by the antibody.

### Determining Binding Affinity

In some embodiments, the antibodies suitable for use with the present invention have a high affinity binding for human GM-CSF or GM-CSF receptor. High affinity binding between an antibody and an antigen exists if the dissociation constant (K_{D}) of the antibody is < 1 nM, and preferably < 100 pM. A variety of methods can be used to determine the binding affinity of an antibody for its target antigen such as surface plasmon resonance assays, saturation assays, or immunoassays such as ELISA or RIA, as are well known to persons of skill in the art. An exemplary method for determining binding affinity is by surface plasmon resonance analysis on a BIAcore™ 2000 instrument (Biacore AB, Freiburg, Germany) using CM5 sensor chips, as described by Krinner et al., (2007) Mol. Immunol. Feb;44(5):916-25. (Epub 2006 May 11)).

### Cell Proliferation Assay for Identifying Neutralizing Antibodies

In some embodiments, the GM-CSF antagonists are neutralizing antibodies to GM-CSF, or its receptor, which bind in a manner that interferes with the binding of GM- CSF. Neutralizing antibodies and other GM-CSF antagonists may be identified using any number of assays that assess GM-CSF function. For example, cell-based assays for GM-CSF receptor signaling, such as assays which determine the rate of proliferation of a GM-CSF-dependent cell line in response to a limiting amount of GM-CSF, are conveniently used. The human TF-1 cell line is suitable for use in such an assay. See, Krinner et al., (2007) Mol. Immunol. In some embodiments, the neutralizing antibodies of the invention inhibit GM-CSF-stimulated TF-1 cell proliferation by at least 50% when a GM-CSF concentration is used which stimulates 90% maximal TF-1 cell proliferation. In other embodiments, the neutralizing antibodies inhibit GM-CSF stimulated proliferation by at least 90%. Thus, typically, a neutralizing antibody, or other GM-CSF antagonist for use in the invention, has an EC₅₀ of less than 10 nM (*e.g.*, Table 1). Additional assays suitable for use in identifying neutralizing antibodies suitable for use with the present invention will be well known to persons of skill in the art.

### Exemplary Antibodies

Antibodies for use in the invention are known in the art and can be produced using routine techniques. Exemplary antibodies are described. It is understood that the exemplary antibodies can be engineered in accordance with the procedures known in the art and summarized herein to produce antibody fragments, chimeras, and the like by either chemical or recombinant technology.

An exemplary chimeric antibody suitable for use as a GM-CSF antagonist is c19/2. The c/19/2 antibody binds GM-CSF with a monovalent binding affinity of about 10pM as determined by surface plasmon resonance analysis. SEQ ID NOs 1 and 2 show the heavy and light chain variable region sequence of c19/2 (*e.g.*, WO03/068920). The CDRs, as defined according to Kabat, are:

| | |
|---|---|
| CDRH1 | DYNIH |
| CDRH2 | YIAPYSGGTGYNQEFKN |
| CDRH3 | RDRFPYYFDY |
| CDRL1 | KASQNVGSNVA |
| CDRL2 | SASYRSG |
| CDRL3 | QQFNRSPLT. |

The CDRs can also be determined using other well known definitions in the art, *e.g.*, Chothia, international ImMunoGeneTics database (IMGT), and AbM.

In some embodiments, an antibody used in the invention competes for binding to, or binds to, the same epitope as c19/2. The GM-CSF epitope recognized by c19/2 has been identified as a product that has two peptides, residues 86-93 and residues 112-127, linked by a disulfide bond between residues 88 and 121. The c19/2 antibody inhibits the GM-CSF-dependent proliferation of a human TF-1 leukemia cell line with an EC₅₀ of 30 pM when the cells are stimulated with 0.5 ng/ml GM-CSF. In some embodiments, the antibody used in the invention binds to the same epitope as c19/2.

An antibody for administration, such as c 19/2, can be additionally humaneered. For example, the c19/2 antibody can be further engineered to contain human V gene segments.

Another exemplary neutralizing anti-GM-CSF antibody is the E10 antibody described in Li et al., (2006) PNAS 103(10):3557-3562. E10 is an IgG class antibody that has an 870 pM binding affinity for GM-CSF. The antibody is specific for binding to human GM-CSF as shown in an ELISA assay, and shows strong neutralizing activity as assessed with a TF1 cell proliferation assay.

An additional exemplary neutralizing anti-GM-CSF antibody is the MT203 antibody described by Krinner et al., (Mol Immunol. 44:916-25, 2007; Epub 2006 May 112006). MT203 is an IgG1 class antibody that binds GM-CSF with picomolar affinity. The antibody shows potent inhibitory activity as assessed by TF-1 cell proliferation assay and its ability to block IL-8 production in U937 cells.

Additional antibodies suitable for use with the present invention will be known to persons of skill in the art.

GM-CSF antagonists that are anti-GM-CSF receptor antibodies can also be employed in the invention. Such GM-CSF antagonists include antibodies to the GM-CSF receptor alpha chain or beta chain. An anti-GM-CSF receptor antibody employed in the invention can be in any antibody format as explained above, *e.g*., intact, chimeric, monoclonal, polyclonal, antibody fragment, humanized, humaneered, and the like. Examples of anti-GM-CSF receptor antibodies, *e.g.*, neutralizing, high-affinity antibodies, suitable for use in the invention are known (*see., e.g.,* US Patent 5,747,032 and Nicola et al., Blood 82: 1724, 1993).

### Non-Antibody GM-CSF Antagonists

Other proteins that may interfere with the productive interaction of GM-CSF with its receptor include mutant GM-CSF proteins and secreted proteins comprising at least part of the extracellular portion of one or both of the GM-CSF receptor chains that bind to GM-CSF and compete with binding to cell-surface receptor. For example, a soluble GM-CSF receptor antagonist can be prepared by fusing the coding region of the sGM-CSFRalpha with the CH2-CH3 regions of murine IgG2a. An exemplary soluble GM-CSF receptor is described by Raines et al. (1991) Proc. Natl. Acad. Sci USA 88: 8203. An example of a GM-CSFRalpha-Fc fusion protein is provided, *e.g.*, in Brown et al (1995) Blood 85: 1488. In some embodiments, the Fc component of such a fusion can be engineered to modulate binding, *e.g.*, to increase binding, to the Fc receptor.

Other GM-CSF antagonist include GM-CSF mutants. For example, GM-CSF having a mutation of amino acid residue 21 of GM-CSF to Arginine or Lysine (E21R or E221K) described by Hercus et al. Proc. Natl. Acad. Sci USA 91:5838, 1994 has been shown to have *in vivo* activity in preventing dissemination of GM-CSF-dependent leukemia cells in mouse xenograft models (Iversen et al. Blood 90:4910, 1997). As appreciated by one of skill in the art, such antagonists can include conservatively modified variants of GM-CSF that have substitutions, such as the substitution noted at amino acid residue 21, or GM-CSF variants that have, *e.g.*, amino acid analogs to prolong half-life.

In other embodiments, the GM-CSF antagonist is an "antibody mimetic" that targets and binds to the antigen in a manner similar to antibodies. Certain of these "antibody mimics" use non-immunoglobulin protein scaffolds as alternative protein frameworks for the variable regions of antibodies. For example, Ku et al. (Proc. Natl. Acad. Sci. U.SA. 92(14):6552-6556 (1995)) discloses an alternative to antibodies based on cytochrome b562 in which two of the loops of cytochrome b562 were randomized and selected for binding against bovine serum albumin. The individual mutants were found to bind selectively with BSA similarly with anti-BSA antibodies.

U.S. Patent Nos. 6,818,418 and 7,115,396 disclose an antibody mimic featuring a fibronectin or fibronectin-like protein scaffold and at least one variable loop. Known as Adnectins, these fibronectin-based antibody mimics exhibit many of the same characteristics of natural or engineered antibodies, including high affinity and specificity for any targeted ligand. The structure of these fibronectin-based antibody mimics is similar to the structure of the variable region of the IgG heavy chain. Therefore, these mimics display antigen binding properties similar in nature and affinity to those of native antibodies. Further, these fibronectin-based antibody mimics exhibit certain benefits over antibodies and antibody fragments. For example, these antibody mimics do not rely on disulfide bonds for native fold stability, and are, therefore, stable under conditions which would normally break down antibodies. In addition, since the structure of these fibronectin-based antibody mimics is similar to that of the IgG heavy chain, the process for loop randomization and shuffling may be employed in vitro that is similar to the process of affinity maturation of antibodies *in vivo.*

Beste et al. (Proc. Natl. Acad. Sci. U.S.A. 96(5):1898-1903 (1999)) disclose an antibody mimic based on a lipocalin scaffold (Anticalin®). Lipocalins are composed of a β-barrel with four hypervariable loops at the terminus of the protein. The loops were subjected to random mutagenesis and selected for binding with, for example, fluorescein. Three variants exhibited specific binding with fluorescein, with one variant showing binding similar to that of an anti-fluorescein antibody. Further analysis revealed that all of the randomized positions are variable, indicating that Anticalin® would be suitable to be used as an alternative to antibodies. Thus, Anticalins® are small, single chain peptides, typically between 160 and 180 residues, which provides several advantages over antibodies, including decreased cost of production, increased stability in storage and decreased immunological reaction.

U.S. Patent No. 5,770,380 discloses a synthetic antibody mimetic using the rigid, non-peptide organic scaffold of calixarene, attached with multiple variable peptide loops used as binding sites. The peptide loops all project from the same side geometrically from the calixarene, with respect to each other. Because of this geometric confirmation, all of the loops are available for binding, increasing the binding affinity to a ligand. However, in comparison to other antibody mimics, the calixarene-based antibody mimic does not consist exclusively of a peptide, and therefore it is less vulnerable to attack by protease enzymes. Neither does the scaffold consist purely of a peptide, DNA or RNA, meaning this antibody mimic is relatively stable in extreme environmental conditions and has a long life span. Further, since the calixarene-based antibody mimic is relatively small, it is less likely to produce an immunogenic response.

Murali et al. (Cell Mol Biol 49(2):209-216 (2003)) describe a methodology for reducing antibodies into smaller peptidomimetics, they term "antibody-like binding peptidomimetics" (ABiP) which may also be useful as an alternative to antibodies.

In addition to non-immunoglobulin protein frameworks, antibody properties have also been mimicked in compounds comprising RNA molecules and unnatural oligomers (e.g., protease inhibitors, benzodiazepines, purine derivatives and beta-turn mimics). Accordingly, non-antibody GM-CSF antagonists can also include such compounds.

### III. Therapeutic Administration

The methods of the invention typically comprise administering methotrexate and a GM-CSF antagonist, (*e.g.*, an anti-GM-CSF antibody) as a pharmaceutical composition to a patient having a chronic inflammatory disease, *e.g.*, RA, in a therapeutically effective amount using a dosing regimen suitable for treatment of the disease.

In some embodiments of the present invention, patients suffering from a chronic inflammatory disease, *e.g.*, RA, are treated with methotrexate at a weekly dose of up to about 25 mg and a GM-CSF antagonist, *e.g.*, an antibody specific for GM-CSF, at a dose that does not induce clinically significant neutropenia and leads to an improvement in one or more markers of inflammation. In some embodiments, the patient response to treatment is determined by showing a significant reduction in the erythrocyte sedimentation rate (ESR) to within the normal range. The normal ESR range is age and gender dependent. For men, normal ESR can be calculated according to the following formula: 0.5x (age in years). For women, normal ESR can be calculated according to the following formula: 0.5 x (age in years +10) (Wallach J. Interpretation of Laboratory Tests, 6th Edition. Little Brown and Company. 1996).

The composition can be formulated for use in a variety of drug delivery systems. One or more physiologically acceptable excipients or carriers can also be included in the compositions for proper formulation. Suitable formulations for use in the present invention are found in Remington's Pharmaceutical Sciences, Mack Publishing Company, Philadelphia, PA, 17th ed. (1985). For a brief review of methods for drug delivery, see, Langer, Science 249: 1527-1533 (1990).

The GM-CSF antagonist for use in the methods of the invention is provided in a solution suitable for injection into the patient such as a sterile isotonic aqueous solution for injection. The GM-CSF antagonist is dissolved or suspended at a suitable concentration in an acceptable carrier. In some embodiments the carrier is aqueous, e.g., water, saline, phosphate buffered saline, and the like. The compositions may contain auxillary pharmaceutical substances as required to approximate physiological conditions, such as pH adjusting and buffering agents, tonicity adjusting agents, and the like.

The pharmaceutical compositions of the invention are administered to a patient suffering from a chronic inflammatory disease, *e.g.*, RA, in an amount sufficient to cure or at least partially arrest the disease or symptoms of the disease and its complications. An amount adequate to accomplish this is defined as a "therapeutically effective dose." A therapeutically effective dose is determined by monitoring a patient's response to therapy. Typical benchmarks indicative of a therapeutically effective dose are known in the art, depending on the disease. For example, for RA, benchmarks include plasma levels of CRP, ESR, blood or urine levels of neopterin, levels of pro-inflammatory cytokines (*e.g.*, TNF-α, GM-CSF, Interleukin-1, Interleukin-6 and Interleukins 8 and 17) or changes in the levels of other pharmacodynamic markers. Other criteria for assessing a therapeutic response can also be used, *e.g.*, by evaluating the number and/or severity of tenderness and swelling of the joints, pain levels, and the like.

Amounts that are administered that are effective will depend upon the severity of the disease and the general state of the patient's health, including other factors such as age, weight, gender, administration route, etc. Single or multiple administrations of the antagonist may be administered depending on the dosage and frequency as required and tolerated by the patient. In any event, the methods provide a sufficient quantity of GM-CSF antagonist in conjunction with methotrexate to effectively treat the patient.

In another embodiment of the invention, the anti-GM-CSF antagonist used to treat a patient suffering from a chronic inflammatory disease such as RA is provided in combination therapy with methotrexate and one or more additional agents, *e.g.*, a nonsteroidal anti-inflammatory agent. Thus, patients may receive additional therapies in order to treat their disease. Such therapies include, but are not limited to, hydroxychloroquinone, sulfasalazine, gold, minocycline, leflunomide, corticosteroids, TNF-antagonists (e.g., etanercept, infliximab or adalimumab), IL-1 antagonists (e.g., as anakinra) or anti-CD20 antibodies (e.g., rituximab). Patients can receive one or more of these additional therapeutic agents as concomitant therapy. Alternatively, patients may be treated sequentially with additional therapeutic agents.

In some embodiments, a patient having a chronic inflammatory disease such as RA is treated with an anti-folate compound other than methotrexate in conjunction with treatment with a GM-CSF antagonist. The anti-folate compound and GM-CSF antagonist are administered in amounts that does not induce clinically significant neutropenia using a dosing regimen suitable for the treatment of the disease.

### A. Administration

The invention provides methods for treatment of patients with chronic inflammatory disease, such as RA, by administering a GM-CSF antagonist in combination with methotrexate. In some embodiments, the GM-CSF antagonist is administered by injection or infusion through any suitable route including but not limited to intravenous, sub-cutaneous, intramuscular or intraperitoneal routes. In an exemplary embodiment, the GM-CSF antagonist is diluted in a physiological saline solution for injection prior to administration to the patient. Such an antagonist is administered, for example, by intravenous infusion over a period of between 15 minutes and 2 hours. In still other embodiments, the administration procedure is via sub-cutaneous or intramuscular injection.

The GM-CSF antagonist is administered while the patient is being treated with methotrexate. In the context of this invention a patient "being treated with methotrexate" or "undergoing treatment with methotrexate" means a patient has been prescribed methotrexate and is therefore receiving, or has recently received, a dose of methotrexate. Typically, methotrexate is taken once a week. Thus, for example, a GM-CSF antagonist can be administered at any time period during the week between doses. In some embodiments, the GM-CSF antagonist can be administered after a patient has received a methotrexate dose, but has not yet taken the next dose, *e.g.*, over a week after the last dose of methotrexate. Such a patient is still considered to be undergoing treatment with methotrexate if the methotrexate therapy is still being prescribed for the patient.

### B. Dosing

The dose of GM-CSF antagonist is chosen in order to provide effective therapy for a patient that has a chronic inflammatory disease. The dose is typically in the range of about 0.1 mg/kg body weight to about 25 mg/kg body weight or in the range about 1 mg to about 2 g per patient. The dose is often in the range of about 1 to about 10 mg/kg or approximately about 50 mg to about 1000 mg / patient. The dose may be repeated at an appropriate frequency which may be in the range once per day to once every three months, depending on the pharmacokinetics of the antagonists (e.g. half-life of the antibody in the circulation) and the pharmacodynamic response (e.g. the duration of the therapeutic effect of the antibody). In some embodiments where the antagonist is an antibody or modified antibody fragment, the *in vivo* half-life of between about 7 and about 25 days and antibody dosing is repeated between once per week and once every 3 months. In other embodiments, the antibody is administered approximately once per month.

Treatment protocols and doses for administering methotrexate are known in the art. For example, recommendations for methotrexate dosing in RA, such as the British Society for Rheumatology's guidelines (July 2000) are 7.5 mg Methotrexate weekly, increasing by 2.5 mg every six weeks to a maximum weekly dose of 25 mg. Dosing for other anti-folate compounds can also be determined using well-know methods.

The anti-folate compound, *e.g.*, methotrexate, and GM-CSF antagonist are administered in a range that does not induce neutropenia. For example, patients receive methotrexate at a dose of up to about 25 mg/week and from about 0.2 to about 10 mg/kig of GM-CSF antagonist.

### EXAMPLES

### Example 1 - Exemplary humaneered antibodies to GM-CSF

A panel of humaneered Fab' molecules with the specificity of c19/2 were generated from epitope-focused human V-segment libraries as described in US patent application 20060134098.

Fab' fragments were expressed from *E. coli.* Cells were grown in 2xYT medium to an OD600 of 0.6. Expression was induced using IPTG for 3 hours at 33°C. Assembled Fab' was obtained from periplasmic fractions and purified by affinity chromatography using Streptococcal Protein G (HiTrap Protein G HP columns; GE Healthcare) according to standard methods. Fab's were eluted in pH 2.0 buffer, immediately adjusted to pH 7.0 and dialyzed against PBS pH7.4.

Binding kinetics were analyzed by Biacore 3000 surface plasmon resonance (SPR). Recombinant human GM-CSF antigen was biotinylated and immobilized on a streptavidin CM5 sensor chip. Fab samples were diluted to a starting concentration of 3 nM and run in a 3 fold dilution series. Assays were run in 10 mM HEPES, 150 mM NaCl, 0.1 mg/mL BSA and 0.005% p20 at pH 7.4 and 37°C. Each concentration was tested twice. Fab' binding assays were run on two antigen density surfaces providing duplicate data sets. The mean affinity (K_{D}) for each of 6 humaneered anti-GM-CSF Fab clones, calculated using a 1:1 Langmuir binding model, is shown in Table 1.

Fabs were tested for GM-CSF neutralization using a TF-1 cell proliferation assay. GM-CSF-dependent proliferation of human TF-1 cells was measured after incubation for 4 days with 0.5 ng/ml GM-CSF using a MTS assay (Cell titer 96, Promega) to determine viable cells. All Fabs inhibited cell proliferation in this assay indicating that these are neutralizing antibodies. There is a good correlation between relative affinities of the anti-GM-CSF Fabs and EC₅₀ in the cell-based assay. Anti-GM-CSF antibodies with monovalent affinities in the range 18 pM -104 pM demonstrate effective neutralization of GM-CSF in the cell-based assay.

**Table 1: Affinity of anti-GM-CSF Fabs determined by surface plasmon resonance analysis in comparison with activity (EC₅₀) in a GM-CSF dependent TF-1 cell proliferation assay**

| **Fab** | **Monovalent binding affinity determined by SPR (pM)** | **EC₅₀(pM) in TF-1 cell proliferation assay** |
|---|---|---|
| 94 | 18 | 165 |
| 104 | 19 | 239 |
| 77 | 29 | 404 |
| 92 | 58 | 539 |
| 42 | 104 | 3200 |
| 44 | 81 | 7000 |

### Example 2 - Exemplary clinical protocol for delivery of anti-GM-CSF antibody

An anti-GM-CSF antibody is stored at 10 mg/ml in sterile isotonic aqueous saline solution for injection at 4°C and is diluted in either 100 ml or 200 ml 0.9% sodium chloride for injection prior to administration to the patient. The antibody is administered to a patient having RA by intravenous infusion over the course of 1 hour at a dose of between 0.2 and 10 mg/kg.

Patients for inclusion in this treatment protocol are chosen based on the following criteria: patients show signs of active RA, patients are currently receiving treatment with methotrexate wherein patients have been receiving stable doses of DMARDs for at least 6 weeks. Furthermore, patients included in this study exhibit the following symptoms: swollen joint count of at least 6 (using 66 joint count), tender joint count of at least 6 (using 68 joint count). At least two of the following criteria are also included in the inclusion criteria: ESR ≥20 mm/hr, CRP ≥15 mg/l, early morning stiffness of ≥45 minutes.

Patients receive either placebo (0.9% sodium chloride for injection) or anti-GM-CSF antibody by intravenous infusion on Day 1 at one of the following doses: 0.2 mg/kg, 1.0 mg/kg, 5.0 mg/kg or 10mg/kg. Patients are monitored for 29 days. All patients continue to receive DMARDs, methotrexate at a dose of up to 25 mg/week, prednisolone up to 10 mg/day, and NSAIDs as clinically appropriate along with medication for any other medical conditions. Throughout the duration of the study the following tests are performed as a study safety assessment: physical examination, vital signs measurement, 12-lead electrocardiogram (ECG), laboratory tests including hematology, biochemistry and urinalysis, pulmonary function tests and incontinence and intensity of adverse events (AEs).

Efficacy of treatment is assessed in two stages. The primary assessment involves an ACR 20 response at any time prior to or at Day 29 of treatment. The secondary assessment includes measuring time to ACR20, proportion of patients who achieve an ACR 50 and 70 response and ESR and CRP measured at Days 8, 15 and 29.

Adverse events, serious adverse events and laboratory abnormalities are tabulated by treatment group and compared to those of the pooled placebo group. The efficacy of the anti-GM-CSF antibody is analyzed by calculating the ACR 20/50/70 responses for intention to treat using a closed testing procedure. The pooled active groups are compared with the patients treated with placebo.

### Example 3 - Treatment of a patient with methotrexate and anti-GM-CSF antibody

A patient that has active RA was treated with methotrexate and an anti-GM-CSF antibody according to the clinical protocol described in Example 2. The patient received 0.2 mg/kg anti-GM-CSF antibody. The patient was also undergoing treatment with 25 mg/week methotrexate.

Blood cell counts were determined by standard methods and included determination of the numbers of: hemoglobin (HGB); total white blood cells (WBCC); platelets (PLT); neutrophils (Neut; also called Absolute Neutrophil Count ANC); lymphocytes (LYMPH); monocytes (MONO); eosinophils (EOSIN); basophils (BASO), hematocrit (HCT). In addition, erythrocyte sedimentation rate (ESR), C-reactive protein (CRP) were determined. The blood cell counts ESR and CRP before and treatment and up to two weeks after treatment are shown in Table 2. As can be seen, after two weeks, the ESR dropped from an abnormal value of 40 to 18, which is within the normal range for an individual of the same sex and age as the treated patient, while the neutrophil count remained unchanged. Accordingly, a combination therapy comprising methotrexate treatment and treatment with an anti-GM-CSF antagonist, in this case an anti-GM-CSF antibody, provided a therapeutic benefit for the treatment of rheumatoid arthritis.

The "1" day in Table 2 indicates when anti-GM-CSF antagonist was administered. The patient had previously been treated with methotrexate and continued receiving methotrexate treatment with the anti-GM-CSF antagonist treatment.

**Table 2. Blood counts and ESR from a patient treated with weekly doses of methotrexate and administered a single dose of anti-GM-CSF antibody on Day 1. The numbers of the various cells (platelets, neutrophils, lymphocytes, etc.) are x 10⁹/L. The ESR is expressed in mm/hr.**

| Days post treatment | HGB | WBCC | PLT | NEUT | LYMPH | HCT | MONO | EOSIN | BASE | ESR |
|---|---|---|---|---|---|---|---|---|---|---|
| -2 | 133 | 5.0 | 202 | 3.04 | 1.26 | 0.4 | 0.52 | 0.16 | 0.02 | 40 |
| 1 | 127 | 4.4 | 208 | 2.64 | 1.23 | 0.38 | 0.36 | 0.15 | 0.03 | |
| 8 | 129 | 5.4 | 189 | 3.01 | 1.41 | 0.39 | 0.76 | 0.17 | 0.04 | 23 |
| 15 | 131 | 4.8 | 193 | 2.76 | 1.39 | 0.39 | 0.46 | 0.15 | 0.04 | 18 |
| 28 | 130 | 5.0 | 180 | 2.79 | 1.71 | 0.4 | 0.25 | 0.21 | 0.04 | 20 |

The above examples are provided by way of illustration only and not by way of limitation. Those of skill in the art will readily recognize a variety of noncritical parameters that could be changed or modified to yield essentially similar results.

All publications, patent applications, accession numbers, and other references cited in this specification are herein incorporated by reference as if each individual publication or patent application were specifically and individually indicated to be incorporated by reference.

### Exemplary sequences

SEQ m NO 1: amino acid sequence for murine 19/2 heavy chain variable region 
Met Glu Leu Ile Met Leu Phe Leu Leu Ser Gly Thr Ala Gly Val His

Ser Glu Val Gln Leu Gln Gln Ser Gly Pro Glu Leu Val Lys Pro Gly

Ala Ser Val Lys Ile Ser Cys Lys Ala Ser Gly Tyr Thr Phe Thr Asp

Tyr Asn Ile His Trp Val Lys Gln Ser His Gly Lys Ser Leu Asp Trp

Ile Gly Tyr Ile Ala Pro Tyr Ser Gly Gly Thr Gly Tyr Asn Gln Glu

Phe Lys Asn Arg Ala Thr Leu Thr Val Asp Lys Ser Ser Ser Thr Ala

Tyr Met Glu Leu Arg Ser Leu Thr Ser Asp Asp Ser Ala Val Tyr Tyr

Cys Ala Arg Arg Asp Arg Phe Pro Tyr Tyr Phe Asp Tyr Trp Gly Gln Gly

Thr Thr Leu Arg Val Ser Ser Val Ser Gly Ser
SEQ ID NO 2: amino acid sequence for murine 19/2 light chain variable region

Met Gly Phe Lys Met Glu Ser Gln Ile Gln Val Phe Val Tyr Met Leu

Leu Trp Leu Ser Gly Val Asp Gly Asp Ile Val Met Ile Gln Ser Gln

Lys Phe Val Ser Thr Ser Val Gly Asp Arg Val Asn Ile Thr Cys Lys

Ala Ser Gln Asn Val Gly Ser Asn Val Ala Trp Leu Gln Gln Lys Pro

Gly Gln Ser Pro Lys Thr Leu Ile Tyr Ser Ala Ser Tyr Arg Ser Gly

Arg Val Pro Asp Arg Phe Thr Gly Ser Gly Ser Gly Thr Asp Phe Ile

Leu Thr Ile Thr Thr Val Gln Ser Glu Asp Leu Ala Glu Tyr Phe Cys

Gln Gln Phe Asn Arg Ser Pro Leu Thr Phe Gly Ser Gly Thr Lys Leu

Glu Leu Lys Arg Ala Asp Ala Ala Pro Thr Val Ser Ile Phe Pro Pro

Ser Ser Lys Gly Glu Phe

### ASPECTS OF THE INVENTION

1. A method for treating a patient suffering from a chronic inflammatory disease, the method comprising administering an anti-folate compound and administering a GM-CSF antagonist to the patient, wherein the anti-folate compound and the GM-CSF antagonist are provided in an amount sufficient to reduce the symptoms of the chronic inflammatory disease, but in an amount that does not induce neutropenia.
2. The method of aspect 1, wherein the anti-folate compound is methotrexate.
3. The method of aspect 1, wherein the chronic inflammatory disease is rheumatoid arthritis.
4. The method of aspect 1, wherein the GM-CSF antagonist is an anti-GM-CSF antibody.
5. The method of aspect 4, wherein the antibody is a polyclonal antibody.
6. The method of aspect 4, wherein the antibody is a monoclonal antibody.
7. The method of aspect 4, wherein the antibody is an antibody fragment that is a Fab, a Fab', a F(ab')₂, a scFv, or a dAB.
8. The method of aspect 7, wherein the antibody fragment is conjugated to polyethylene glycol.
9. The method of aspect 4, wherein the antibody has an affinity ranging from about 5 pM to about 50 pM.
10. The method of aspect 4, wherein the antibody is a neutralizing antibody.
11. The method of aspect 4, wherein the antibody is a recombinant or chimeric antibody.
12. The method of aspect 4, wherein the antibody is a human antibody.
13. The method of aspect 4, wherein the antibody comprises a human variable region.
14. The method of aspect 4, wherein the antibody comprises a human light chain constant region.
15. The method of aspect 4, wherein the antibody comprises a human heavy chain constant region.
16. The method of aspect 15, wherein the human heavy chain constant region is a gamma chain.
17. The method of aspect 4, wherein the antibody binds to the same epitope as chimeric 19/2.
18. The method of aspect 4, wherein the antibody comprises the V_{H} and V_{L} regions of chimeric 19/2.
19. The method of aspect 18, wherein the antibody comprises a human heavy chain constant region.
20. The method of aspect 19, wherein the human heavy chain constant region is a gamma region.
21. The method of aspect 4, wherein the antibody comprises the V_{H} region and V_{L} region CDR1, CDR2, and CDR3 of chimeric 19/2.
22. The method of aspect 4, wherein the antibody comprises the V_{H} region CDR3 and V_{L} region CDR3 of chimeric 19/2.
23. The method of aspect 1, wherein the GM-CSF antagonist is selected from the group consisting of an anti-GM-CSF receptor antibody, a soluble GM-CSF receptor, a cytochrome b562 antibody mimetic, an adnectin, a lipocalin scaffold antibody mimetic, a calixarene antibody mimetic, and an antibody like binding peptidomimetic.
24. A method for treating a patient suffering from rheumatoid arthritis, the method comprising administering methotrexate and administering a therapeutically effective amount of an anti-GM-CSF antibody, wherein the anti-GM-CSF antibody comprises a humaneered Fab' with the binding specificity of chimeric 19/2 and has an affinity ranging from about 5 to about 50 pM.
25. A method for treating a patient suffering from Alzheimer's disease, the method comprising administering a therapeutically effective amount of anti-GM-CSF antibody to the patient.
26. Use of an anti-GMCSF antibody in a therapeutically effective amount for the preparation of a medicament for the treatment of chronic inflammatory disease in a patient undergoing treatment with an anti-folate compound, wherein the therapeutically effective amount is sufficient to reduce the symptoms of chronic inflammatory disease, but does not induce neutropenia.
27. The use of aspect 26, wherein the anti-folate compound is methotrexate.
28. The use of aspect 26 or aspect 27, wherein the antibody is a monoclonal antibody.
29. The use of any one of aspects 26 to 28, wherein the antibody is a recombinant or chimeric antibody.
30. The use of any one of aspects 26 to 29, wherein the antibody comprises a human variable region.
31. The use of any one of aspects 26 to 30, wherein the antibody comprises a human light chain constant region.
32. The use of any one of aspects 26 to 31, wherein the antibody comprises a human heavy chain constant region.
33. The use of aspect 32, wherein the human heavy chain constant region is a gamma region.
34. The use of any one of aspects 26 to 33, wherein the antibody binds to the same epitope as chimeric 19/2.
35. The use of any one of aspects 26 to 34, wherein the antibody comprises the V_{H} region CDR3 and V_{L} region CDR3 of chimeric 19/2.
36. The use of any one of aspects 26 to 35, wherein the antibody comprises the V_{H} region and V_{L} region CDR1, CDR2, and CDR3 of chimeric 19/2.
37. The use of any one of aspects 26 to 36, wherein the antibody comprises the V_{H} and V_{L} regions of chimeric 19/2.
38. The use of any one of aspects 26 to 37, wherein the antibody is an antibody fragment that is a Fab, a Fab', a F(ab')₂, a scFv, or a dAB.
39. The use of any one of aspects 26 to 38, wherein the antibody has an affinity ranging from about 5 pM to about 50 pM.
40. The use of any one of aspects 26 to 34, wherein the antibody is a human antibody.
41. The use of any one of aspects 26 to 40, wherein the antibody is a polyclonal antibody.
42. The use of any one of aspects 26 to 41, wherein the antibody is a neutralizing antibody.
43. Use of a therapeutically effective amount of an anti-GM-CSF antibody comprising a humaneered Fab' with the binding specificity of chimeric 19/2 that has an affinity ranging from about 5 to about 50 pM for the preparation of a medicament for the treatment of a chronic inflammatory disease in a patient undergoing treatment with methotrexate, wherein the therapeutically effective amount is sufficient to reduce the symptoms of chronic inflammatory disease, but does not induce neutropenia.
44. The use of any one of aspects 26 to 43, wherein the antibody is conjugated to polyethylene glycol.
45. Use of an GMCSF antagonist in a therapeutically effective amount for the preparation of a medicament for the treatment of chronic inflammatory disease in a patient undergoing treatment with an anti-folate compound, wherein the therapeutically effective amount is sufficient to reduce the symptoms of chronic inflammatory disease, but does not induce neutropenia.
46. The use of aspect 45, wherein the anti-folate compound is methotrexate.
47. The use of aspect 45 or aspect 46, wherein the GM-CSF antagonist is selected from an anti-GM-CSF receptor antibody; a soluble GM-CSF receptor; a cytochrome b562 antibody mimetic; an adnectin; a lipocalin scaffold antibody mimetic; a calixarene antibody mimetic; or an antibody like binding peptidomimetic.
48. The use of any one of aspects 26 to 47, wherein the chronic inflammatory disease is rheumatoid arthritis.
49. Use of a therapeutically effective amount of an anti-GM-CSF antibody for the preparation of a medicament for the treatment of a patient suffering from Alzheimer's disease.

## Claims

1. A GM-CSF antagonist for use in a method for treating a chronic inflammatory disease in a patient undergoing treatment with an anti-folate compound, wherein the anti-folate compound and the GM-CSF antagonist are provided in an amount sufficient to reduce the symptoms of the chronic inflammatory disease but in an amount that does not induce neutropenia.

2. The GM-CSF antagonist of claim 1 for use in said method wherein the anti-folate compound is methotrexate.

3. The GM-CSF antagonist of claim 1 for use in said method wherein the chronic inflammatory disease is rheumatoid arthritis.

4. The GM-CSF antagonist of claim 1 that is an anti-GM-CSF antibody.

5. The GM-CSF antagonist of claim 4, wherein the antibody
(a) is a polyclonal antibody;
(b) is a monoclonal antibody;
(c) is an antibody fragment that is a Fab, a Fab', a F(ab')₂, a scFv, or a dAB;
(d) has an affinity ranging from about 5 pM to about 50 pM;
(e) is a neutralizing antibody;
(f) is a recombinant or chimeric antibody;
(g) is a human antibody;
(h) comprises a human variable region;
(i) comprises a human light chain constant region;
(j) comprises a human heavy chain constant region;
(k) binds to the same epitope as chimeric 19/2;
(l) comprises the V_{H} and V_{L} regions of chimeric 19/2;
(m) comprises the V_{H} region and V_{L} region CDR1, CDR2, and CDR3 of chimeric 19/2; or
(n) comprises the V_{H} region CDR3 and V_{L} region CDR3 of chimeric 19/2.

6. The GM-CSF antagonist antibody of claim 4, wherein the antibody is an antibody fragment that is a Fab, Fab', F(ab')₂, scFv, or dAB and is conjugated to polyethylene glycol.

7. The GM-CSF antagonist of claim 4, wherein the antibody comprises a human heavy chain constant region that is a gamma region.

8. The GM-CSF antagonist of claim 4, wherein the antibody comprises the V_{H} and the V_{L} regions of chimeric 19/2 and a human heavy chain constant region.

9. The GM-CSF antagonist of claim 8, wherein the human heavy chain constant region is a gamma region.

10. The GM-CSF antagonist of claim 1, wherein the GM-CSF antagonist is selected from the group consisting of an anti-GM-CSF receptor antibody, a soluble GM-CSF receptor, a cytochrome b562 antibody mimetic, an adnectin, a lipocalin scaffold antibody mimetic, a calixarene antibody mimetic, and an antibody-like binding peptidomimetic.

11. The GM-CSF antagonist of claim 1 for use in said method wherein the anti-folate compound is methotrexate and the GM-CSF antagonist is an anti-GM-CSF antibody, wherein the anti-GM-CSF antibody comprises a humaneered Fab' with the binding specificity of chimeric 19/2 and has an affinity ranging from about 5 to about 50 pM.

12. The GM-CSF antagonist of claim 11 for use in said method wherein the chronic inflammatory disease is rheumatoid arthritis.

13. An anti-GM-CSF antibody for use in a method for treating a patient suffering from Alzheimer's disease.

14. Use of a GM-CSF antagonist in a therapeutically effective amount for the preparation of a medicament for the treatment of chronic inflammatory disease in a patient undergoing treatment with an anti-folate compound, wherein the therapeutically effective amount is sufficient to reduce the symptoms of chronic inflammatory disease, but does not induce neutropenia.

15. The use of claim 14, wherein
(a) the anti-folate compound is methotrexate;
(b) the GM-CSF antagonist is selected from an anti-GM-CSF antibody; an anti-GM-CSF receptor antibody; a soluble GM-CSF receptor; a cytochrome b562 antibody mimetic; an adnectin, a lipocalin scaffold antibody mimetic, a calixarene antibody mimetic, and an antibody-like binding peptidomimetic; or
(c) the chronic inflammatory disease is rheumatoid arthritis.


SEQUENCE LISTING

<110> Bebbington, Christopher R.
      Yarranton, Geoffrey T.
      KaloBios Pharmaceuticals, Inc.

<120> Methods of Treating Chronic Inflammatory
  Diseases Using a GM-CSF Antagonist

<130> 021167-002520PC

<140> WO PCT/US07/85407
<141> 2007-11-21

<150> US 60/860,780
<151> 2006-11-21

<150> US 60/902,742
<151> 2007-02-21

<160> 8

<170> FastSEQ for Windows Version 4.0

<210> 1
<211> 140
<212> PRT
<213> Artificial Sequence

<220>
<223> murine chimeric anti-GM-CSF antagonist antibody
      c19/2 heavy chain varable region

<400> 1
Met Glu Leu Ile Met Leu Phe Leu Leu Ser Gly Thr Ala Gly Val His
 1               5                   10                  15
Ser Glu Val Gln Leu Gln Gln Ser Gly Pro Glu Leu Val Lys Pro Gly
            20                  25                  30
Ala Ser Val Lys Ile Ser Cys Lys Ala Ser Gly Tyr Thr Phe Thr Asp
        35                  40                  45
Tyr Asn Ile His Trp Val Lys Gln Ser His Gly Lys Ser Leu Asp Trp
    50                  55                  60
Ile Gly Tyr Ile Ala Pro Tyr Ser Gly Gly Thr Gly Tyr Asn Gln Glu
65                  70                  75                  80
Phe Lys Asn Arg Ala Thr Leu Thr Val Asp Lys Ser Ser Ser Thr Ala
                85                  90                  95
Tyr Met Glu Leu Arg Ser Leu Thr Ser Asp Asp Ser Ala Val Tyr Tyr
            100                 105                 110
Cys Ala Arg Arg Asp Arg Phe Pro Tyr Tyr Phe Asp Tyr Trp Gly Gln
        115                 120                 125
Gly Thr Thr Leu Arg Val Ser Ser Val Ser Gly Ser
    130                 135                 140

<210> 2
<211> 150
<212> PRT
<213> Artificial Sequence

<220>
<223> murine chimeric anti-GM-CSF antagonist antibody
      c19/2 light chain varable region

<400> 2
Met Gly Phe Lys Met Glu Ser Gln Ile Gln Val Phe Val Tyr Met Leu
 1               5                  10                  15
Leu Trp Leu Ser Gly Val Asp Gly Asp Ile Val Met Ile Gln Ser Gln
            20                  25                  30
Lys Phe Val Ser Thr Ser Val Gly Asp Arg Val Asn Ile Thr Cys Lys
        35                  40                  45
Ala Ser Gln Asn Val Gly Ser Asn Val Ala Trp Leu Gln Gln Lys Pro
    50                  55                  60
Gly Gln Ser Pro Lys Thr Leu Ile Tyr Ser Ala Ser Tyr Arg Ser Gly
65                  70                  75                  80
Arg Val Pro Asp Arg Phe Thr Gly Ser Gly Ser Gly Thr Asp Phe Ile
                85                  90                  95
Leu Thr Ile Thr Thr Val Gln Ser Glu Asp Leu Ala Glu Tyr Phe Cys
            100                 105                 110
Gln Gln Phe Asn Arg Ser Pro Leu Thr Phe Gly Ser Gly Thr Lys Leu
        115                 120                 125
Glu Leu Lys Arg Ala Asp Ala Ala Pro Thr Val Ser Ile Phe Pro Pro
    130                 135                 140
Ser Ser Lys Gly Glu Phe
145                 150


<210> 3
<211> 5
<212> PRT
<213> Artificial Sequence

<220>
<223> murine chimeric anti-GM-CSF antagonist antibody
      c19/2 heavy chain varable region CDRH1

<400> 3
Asp Tyr Asn Ile His
 1               5


<210> 4
<211> 17
<212> PRT
<213> Artificial Sequence

<220>
<223> murine chimeric anti-GM-CSF antagonist antibody
      c19/2 heavy chain varable region CDRH2

<400> 4
Tyr Ile Ala Pro Tyr Ser Gly Gly Thr Gly Tyr Asn Gln Glu Phe Lys
 1               5                  10                  15
Asn


<210> 5
<211> 10
<212> PRT
<213> Artificial Sequence

<220>
<223> murine chimeric anti-GM-CSF antagonist antibody
      c19/2 heavy chain varable region CDRH3

<400> 5
Arg Asp Arg Phe Pro Tyr Tyr Phe Asp Tyr
 1               5                   10

<210> 6
<211> 11
<212> PRT
<213> Artificial Sequence

<220>
<223> murine chimeric anti-GM-CSF antagonist antibody
      c19/2 light chain varable region CDRL1

<400> 6
Lys Ala Ser Gln Asn Val Gly Ser Asn Val Ala
 1               5                   10

<210> 7
<211> 7
<212> PRT
<213> Artificial Sequence

<220>
<223> murine chimeric anti-GM-CSF antagonist antibody
      c19/2 light chain varable region CDRL2

<400> 7
Ser Ala Ser Tyr Arg Ser Gly
 1               5

<210> 8
<211> 9
<212> PRT
<213> Artificial Sequence

<220>
<223> murine chimeric anti-GM-CSF antagonist antibody
      c19/2 light chain varable region CDRL3

<400> 8
Gln Gln Phe Asn Arg Ser Pro Leu Thr
 1               5